# EUROPEAN PATENT APPLICATION

(11) **EP 4 176 902 A1**
(43) Date of publication of application: **10.05.2023**
(21) Application number: 21834289.7
(22) Date of filing: 01.07.2021
(51) Int. Cl.: A61K 47/32, A61K 9/10, A61K 9/16, A61K 31/4439, A61K 31/496, A61K 31/517, A61K 31/519, A61K 31/5377, A61K 31/553, A61K 45/00, A61K 47/02, A61K 47/04, A61K 47/12, A61K 47/18, A61K 47/22, A61K 47/34, A61K 47/38, A61P 9/00, A61P 35/00

(54) **ORAL PHARMACEUTICAL COMPOSITION AND METHOD FOR PRODUCING SAME**

(30) Priority: 02.07.2020 JP 2020114978; 28.01.2021 JP 2021011901
(71) Applicant: Artham Therapeutics Inc., Kanagawa 231-0023 (JP)
(72) Inventor: TANAKA, Akira, Yokohama-shi, Kanagawa 231-0023 (JP); FUNATANI, Chiaki, Saitama-shi, Saitama 331-0046 (JP); KURA, Kenichi, Saitama-shi, Saitama 331-0046 (JP); KIMURA, Naoki, Saitama-shi, Saitama 331-0046 (JP)
(74) Representative: Jones, Nicholas Andrew
(86) International application number: PCT/JP2021/025019
(87) International publication number: WO 2022/004859

(57) **Abstract**

Provided is a pharmaceutical composition for orally administering a pharmaceutical active ingredient which has a pH-dependent solubility profile, said pharmaceutical composition making it possible to achieve excellent scrubbing efficiency regardless of the pH inside the alimentary canal. Said oral pharmaceutical composition contains a pharmaceutical active ingredient which has a pH-dependent solubility profile, an inorganic or organic acid, and a hydrophilic polymer.

## Description

### TECHNICAL FIELD

The present invention relates to an oral pharmaceutical composition for administering an active pharmaceutical ingredient having a pH-dependent dissolution profile as well as a method for producing the same.

### BACKGROUND ART

Pharmaceutical compositions for oral administration of active pharmaceutical ingredients should preferably exhibit certain dissolution characteristics to achieve stable absorption of active pharmaceutical ingredients by the digestive tract of the subject. However, the pH in the digestive tract is not constant. In particular, the pH in the stomach varies widely among individuals, and there are large fluctuations even within the same individual, depending on conditions such as physical conditions and dietary contents. It is therefore extremely difficult to orally administer active pharmaceutical ingredients, especially those with pH-dependent dissolution profiles, so as to achieve stable absorption.

Examples of active pharmaceutical ingredients having pH-dependent dissolution profiles include ART-001 (compound name: serabelisib), which is currently under development by the applicant. The chemical structure of ART-001 is shown below. ART-001 is currently undergoing clinical trials as an active pharmaceutical ingredient that has therapeutic effects on diseases such as vascular malformation. 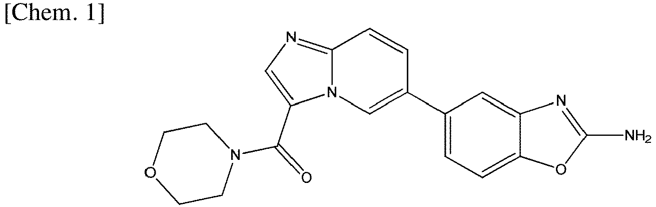 ART-001 (serabelisib)

ART-001 has been reported to have a pH-dependent dissolution profile, showing high solubility in acidic solutions but extremely low solubility in neutral to basic solutions (Non-Patent Literature 1). In a previous Phase I clinical study carried out for patients with advanced solid tumors, ART-001 showed a high degree of variability in pharmacokinetics and limited therapeutic efficacy (Non-Patent Literature 2). ART-001 also exhibited significant pharmacokinetic variability in pharmacokinetic studies in healthy adults (Non-Patent Literature 1). Furthermore, in the same study, when gastric acid secretion was suppressed by co-administration of a proton pump inhibitor (lansoprazole) to thereby adjust the pH in the stomach to neutral, absorption of ART-001 was markedly decreased. These findings suggest that changes in intragastric pH may be related to changes in the solubility and absorption of ART-001 in the digestive tract. Also, pH-dependent solubility and variations in pharmacokinetics in clinical studies have been observed for alperisib, which has a similar mechanism of action to ART-001, (Non-Patent Literature 3 and 4). Thus, in order to maintain stable pharmacokinetics and express therapeutic effects of poorly-soluble active pharmaceutical ingredients with pH-dependent dissolution profiles, it is desired to develop a formulation that can be stably absorbed regardless of the pH in the stomach.

### LIST OF CITATIONS

### Non-Patent Literature

[Non-Patent Literature 1] Clinical Pharmacology in Drug Development, 2019, Vol.8, No.5, pp.637-646
[Non-Patent Literature 2] Clinical Cancer Research, 2017, Vol.23, No.17, pp.5218-5224
[Non-Patent Literature 3] FDA / CENTER FOR DRUG EVALUATION AND RESEARCH, APPLICATION NUMBER: 212526Orig1s000 PRODUCT QUALITY REVIEW(S) (approval date: May 24, 2019)
[Non-Patent Literature 4] FDA / CENTER FOR DRUG EVALUATION AND RESEARCH, APPLICATION NUMBER: 212526Orig1s000 MULTI-DISCIPLINE REVIEW

### SUMMARY OF INVENTION

### PROBLEM TO BE SOLVED

The present invention was made in view of the above problem, and is intended to provide a pharmaceutical composition for oral administration of an active pharmaceutical ingredient that has a pH-dependent dissolution profile and is capable of achieving excellent absorption efficiency regardless of the pH in the digestive tract.

### MEANS TO SOLVE THE PROBLEM

The present inventors have made thorough investigation and, as a result, have founded that a pharmaceutical composition that can achieve excellent absorption efficiency regardless of the pH in the digestive tract can be obtained by formulating an active pharmaceutical ingredient having such a pH-dependent dissolution profile together with an inorganic or organic acid and a hydrophilic polymer, and have thereby completed the present invention.

Specifically, aspects of the present invention include the following:
[Aspect 1] An oral pharmaceutical composition comprising: an active pharmaceutical ingredient having a pH-dependent dissolution profile; an inorganic or organic acid; and a hydrophilic polymer.
[Aspect 2] The oral pharmaceutical composition according to Aspect 1, wherein the active pharmaceutical ingredient is an aromatic compound having a π-conjugated system.
[Aspect 3] The oral pharmaceutical composition according to Aspect 1 or 2, wherein the active pharmaceutical ingredient is a compound having a structure represented by Formula I. 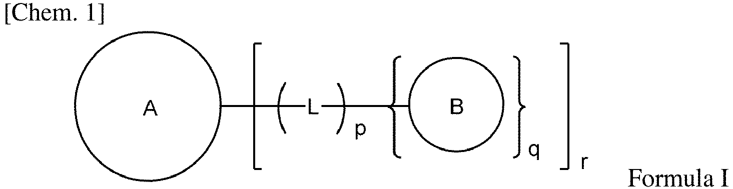 where
   Ring A represents a 5- to 16-membered monocyclic or fused bicyclic or tricyclic aromatic hydrocarbon group or aromatic heterocyclic group that may have one or more substituents, Ring B represents a 5- to 12-membered monocyclic or fused bicyclic aromatic hydrocarbon group or aromatic heterocyclic group that may have one or more substituents, L represents a divalent or trivalent linking group that may have one or more substituents, p represents an integer of 0 or 1,
   q represents an integer of 1 or 2, and
   r represents an integer of 1 or 2.
[Aspect 4] The oral pharmaceutical composition according to any one of Aspects 1 to 3, wherein the active pharmaceutical ingredient is one or more compounds selected from ART-001 (serabelisib), alpelisib, afatinib, gefitinib, bosutinib, alectinib, palbociclib, taselisib, and copanlisib .
[Aspect 5] The oral pharmaceutical composition according to any one of Aspects 1 to 4, wherein the ratio of the solubility at pH 6 to the solubility at pH 2.5 of the active pharmaceutical ingredient is 50% or less, or 30% or less, or 20% or less, or 10% or less, or 5% or less..
[Aspect 6] The oral pharmaceutical composition according to any one of Aspects 1 to 5, wherein the inorganic or organic acid is one or more acids selected from phosphoric acid, hydrochloric acid, citric acid, malic acid, tartaric acid, ascorbic acid, fumaric acid, succinic acid, aspartic acid, lactic acid, acetic acid, glutamic acid, and adipic acid.
[Aspect 7] The oral pharmaceutical composition according to any one of Aspects 1 to 6, wherein the hydrophilic polymer is one or more polymers selected from polyvinyl alcohol, povidone, hypromellose, copovidone, hydroxypropylcellulose, polyvinyl alcohol-polyethylene glycol-graft copolymer, hypromellose phthalate, and hypromellose acetate succinate.
[Aspect 8] The oral pharmaceutical composition according to any one of Aspects 1 to 7, wherein the content percentage of the active pharmaceutical ingredient is 2 mass % or more, or 5 mass % or more, or 10 mass % or more, or 15 mass % or more, or 40 mass % or less, or 35 mass % or less, or 30 mass % or less, or 25 mass % or less.
[Aspect 9] The oral pharmaceutical composition according to any one of Aspects 1 to 8, wherein the content ratio of the inorganic or organic acid to one mass part of the active pharmaceutical ingredient is 0.1 mass parts or more, or 0.3 mass parts or more, or 0.5 mass parts or more, or 10 mass parts or less, or 7.5 mass parts or less, or 5 mass parts or less.
[Aspect 10] The oral pharmaceutical composition according to any one of Aspects 1 to 9, wherein the content ratio of the hydrophilic polymer to one mass part of the active pharmaceutical ingredient is 0.02 mass parts or more, or 0.05 mass parts or more, or 0.1 mass parts or more, or 5 mass parts or less, or 3 mass parts or less, or 2 mass parts or less..
[Aspect 11] The oral pharmaceutical composition according to any one of Aspects 1 to 10, which is in the form of a dry syrup.
[Aspect 12] A method of producing an oral pharmaceutical composition according to any one of Aspects 1 to 11, comprising: wet-granulating a mixture containing the active pharmaceutical ingredient, the inorganic or organic acid, and the hydrophilic polymer, and drying the granulated product.
[Aspect 13] An oral pharmaceutical composition obtainable by the method according to Aspect 12.

### EFFECT OF THE INVENTION

According to an aspect of the oral composition of the present invention, when an active pharmaceutical ingredient with a pH-dependent dissolution profile is orally administered, excellent absorption efficiency can be achieved regardless of the pH in the digestive tract. In addition, according to an aspect of the oral composition of the present invention, more stable pharmacokinetics with less inter-individual variability can be achieved compared to previously-reported dosage forms such as capsules or tablets.

### EXPLANATION OF FIGURES

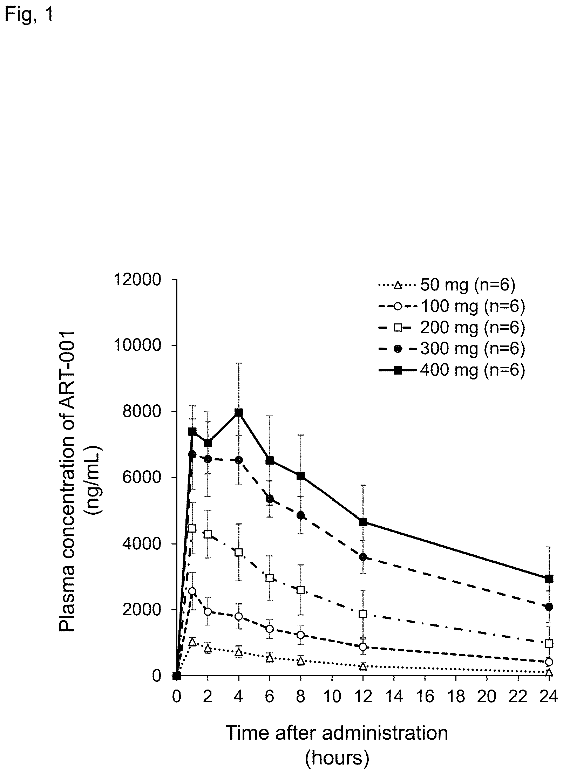

[Figure 1] Figure 1 is a graph showing the plasma concentration of ART-001 over time after a single administration of a dry syrup formulation of ART-001 (the pharmaceutical composition of the present invention) to a healthy adult. The plots indicate means ± standard deviations.

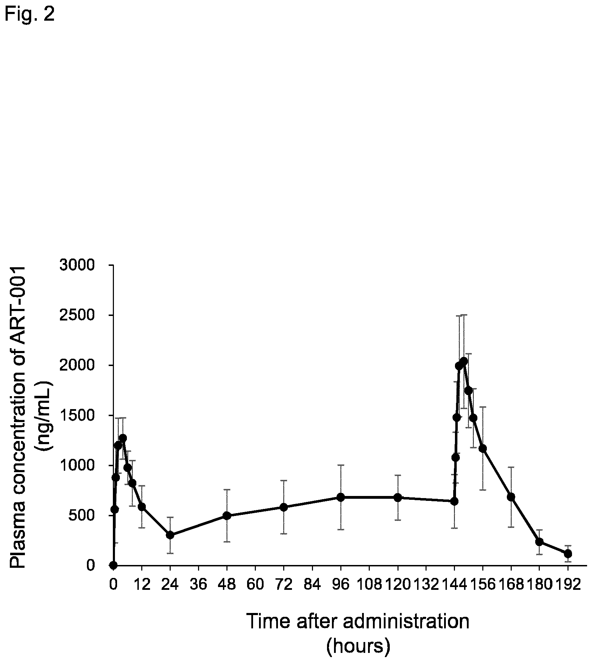

[Figure 2] Figure 2 is a graph showing the plasma concentration of ART-001 over time after repeated administration of a dry syrup formulation of ART-001 (the pharmaceutical composition of the present invention) to healthy adults for 7 days. From day 2 to day 6 of the administration period, blood samples were collected only 24 hours after the previous day's administration. The plots indicate means ± standard deviations.

### EMBODIMENTS

The present invention will be described in detail in accordance with the specific embodiments below. However, the present invention should not be restricted by the following embodiments, but can be implemented in any form to the extent that it does not depart from the purpose of the present invention. In addition, a combination of any two or more of the various forms described below shall also be included in the scope of the present invention, except when clearly contradicted by the definition or context.

### [Oral Pharmaceutical Composition]

An embodiment of the present invention relates to a pharmaceutical composition for orally administering an active pharmaceutical ingredient having a pH-dependent dissolution profile (also referred to as "the pharmaceutical composition of the present invention"). In addition to the active pharmaceutical ingredient having such a pH-dependent dissolution profile, the pharmaceutical composition of the present invention also contains an inorganic or organic acid and a hydrophilic polymer.

According to the pharmaceutical composition of the present invention having these components, excellent absorption efficiency can be achieved regardless of the pH in the digestive tract when used for oral administration of active pharmaceutical ingredients with pH-dependent dissolution profiles. In fact, the present inventor have shown that when the pharmaceutical compositions of the present invention were used to administer active pharmaceutical ingredients with pH-dependent dissolution profiles, excellent absorption efficiency was achieved regardless of the pH in the digestive tract in animal administration tests, as shown in the examples described below (see "Example 3. Evaluation of ART-001 dry syrup formulations"). The present inventor have also shown in human dosing studies that in comparison to previously-reported capsules or tablets (see Juric et al., Clin. Cancer Res., (2017), 23[17]:5015-5023 and Patel et al., Clin. Pharmacol. Drug Dev, (2019), 8[5]:637-646), the pharmaceutical compositions of the present invention achieved more stable pharmacokinetics with less inter-individual variability (see "Example 4. Study of blood pharmacokinetics of ART-001 dry syrup formulations"). The tight and stable pharmacokinetics obtained by such dry syrup formulations may facilitate fine dose selection to obtain the expected effective blood exposure, and may also serve to avoid side effects due to high exposure.

### Active pharmaceutical ingredients having pH-dependent dissolution profiles:

The pharmaceutical composition of the present invention contains an active pharmaceutical ingredient having a pH-dependent dissolution profile.

The term "active pharmaceutical ingredient" used herein means an ingredient to be contained in a pharmaceutical product that exhibits physiological activity for a desired indication. The indications and physiological activities of active pharmaceutical ingredients used herein are not limited, but include active pharmaceutical ingredients that exert their medicinal effects when they are orally administered to a subject and absorbed in the digestive tract (e.g., in the stomach).

The term "pH-dependent dissolution profile" used herein means that the solubility varies with pH. Unless otherwise stated, the terms "dissolution profile" and "solubility" used herein mean solubility in an aqueous medium. The term "aqueous medium" used herein means water or various aqueous solutions. Aqueous digestive fluids (e.g., saliva, gastric juice, etc.) and other aqueous body fluids (blood, lymph fluid, tissue fluid, ascites fluid, etc.) shall also fall under the category of aqueous media.

According to an embodiment, the active pharmaceutical ingredients with pH-dependent dissolution profiles used herein may be, although are not limited to, compounds that exhibit high solubility under acidic pH conditions but less solubility as pH increases, and little solubility under neutral to alkaline pH conditions. More specifically, examples of such active pharmaceutical ingredients include compounds that exhibit, when dissolved in an aqueous solvent at near room temperature (e.g., 30°C), a solubility under neutral pH conditions (e.g., pH 6) which is 50% or less, particularly 30% or less, or 20% or less, or 10% or less, especially 5% or less, of the solubility under acidic pH conditions (e.g., pH 2.5). Accordingly, examples of active pharmaceutical ingredients to be contained in the pharmaceutical composition of the present invention include compounds with basic nitrogen-containing heterocyclic rings and compounds with basic functional groups, such as unsubstituted or substituted amino groups.

The active pharmaceutical ingredients to be contained in the pharmaceutical composition of the present invention are not limited as long as they have pH-dependent dissolution profiles. Examples of such active pharmaceutical ingredients include compounds that can form stable crystal structures. Specific examples of such active pharmaceutical ingredients include aromatic compounds that have a π-conjugated system and can form π-π stacking. The term "π-π stacking" used herein means the phenomenon in which two or more aromatic compound molecules are stacked on a plane and stabilized by π-π interactions. Aromatic compounds that can produce π-π stacking can generally form stable crystal structures and have low solubility in aqueous media. Application of the present invention to such aromatic compounds may lead to a remarkable improvement in solubility profiles.

According to an embodiment, examples of the active pharmaceutical ingredients to be contained in the pharmaceutical compositions of the present invention are aromatic compounds with a large π-conjugated system, e.g., compounds with fused aromatic rings and/or interlinked multiple aromatic rings. Such compounds are prone to π-π stacking, which in turn leads to crystallization and deterioration of solubility. Accordingly, application of the pharmaceutical composition of the present invention is applied to such compounds may result in a more pronounced effect on improving the solubility profiles. Specific examples of such active pharmaceutical ingredients include, although not limited to, compounds having a structure represented by Formula I below.

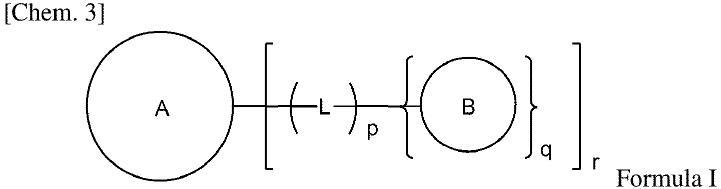

In Formula I, Ring A represents a 5- to 16-membered monocyclic or fused bicyclic or tricyclic aromatic hydrocarbon group or aromatic heterocyclic group that may have one or more substituents.

In Formula I, Ring B represents a 5- to 12-membered monocyclic or fused bicyclic aromatic hydrocarbon group or aromatic heterocyclic group that may have one or more substituents.

In Formula I, examples of aromatic hydrocarbon groups as Ring A and/or Ring B include, although not limited to, groups selected from the following list. (This list indicates the names of monovalent groups. When r is 2, Ring A may be selected from divalent groups obtained by removing one more hydrogen radical from the monovalent groups below.)
*(Monocyclic group) Phenyl group.
*(Fused bicyclic groups) Indenyl, naphthyl, and azulenyl groups.
*(Fused tricyclic groups) Anthracenyl, phenanthrenyl, and fluorenyl groups.

In Formula I, aromatic heterocyclic groups as Ring A and/or Ring B include one or more hetero atoms selected from nitrogen atom, oxygen atom, and sulfur atom. Examples of aromatic heterocyclic groups as Ring A and/or Ring B include, although not limited to, groups selected from the following list. (This list indicates the names of monovalent groups. When r is 2, Ring A may be selected from divalent groups obtained by removing one more hydrogen radical from the monovalent groups below.)
*(Monocyclic groups) Pyrrolyl, pyrazolyl, imidazolyl, triazolyl, furanyl, thiophenyl, oxazolyl, isoxazolyl, thiazolyl, oxadiazolyl, thiadiazolyl, pyridinyl, pyridazinyl, pyrimidinyl, pyrazinyl, triazinyl, and pyranyl groups.
*(Fused bicyclic groups) Indenyl, indolyl, isoindolyl, indolizinyl, indazolyl, benzoimidazolyl, azaindolyl, azaindazolyl, pyrazolopyrimidinyl, purinyl, benzofuranyl, isobenzofuranyl, benzothiophenyl, benzoxazolyl, benzothiazolyl, benzoisoxazolyl, benzoisothiazolyl, quinolinyl, isoquinolinyl, quinolizinyl, quinoxalinyl, phthalazinyl, quinazolinyl, naphthyridinyl, pyridopyrimidinyl, pyridopyrazinyl, and benzopyranyl groups.
*(Fused tricyclic groups) Carbazolyl, dibenzofuranyl, acridinyl, phenazinyl, phenoxazinyl, phenothiazinyl, phenoxathiinyl groups.

In Formula I, L represents a divalent or trivalent linking group that may have one or more substituents. Specifically, when q mentioned below is 1, L is a divalent linking group, and when q is 2, L is a trivalent linking group. The linking groups are not limited, but may preferably be groups that can be conjugated with Ring A and/or Ring B.

In Formula I, examples of linking groups L include, although not limited to, groups selected from the list below.
*Divalent or trivalent groups obtained by removing one or two more hydrogen radicals from alkyls (methyl, ethyl, propyl (n-propyl and isopropyl), butyl (n-butyl, sec-butyl, isobutyl, and tert-butyl), pentyl, hexyl, heptyl, octyl, nonyl, and decyl), alkenyls, or alkynyls. The number of carbon atoms in such divalent or trivalent groups are not particularly limited, but may be from 1 to 10, preferably from 1 to 7, more preferably from 1 to 5.
*Carbonyl group.
*Azo group, diazo group, divalent or trivalent groups obtained by removing one or two more hydrogen radicals from (primary, secondary, or tertiary) amino groups, or amide groups.
*Sulfide, sulfinyl, and sulfonyl groups.
*Oxy and dioxy groups.

Examples of linking groups also include groups consisting of any two or more of the aforementioned linking groups connected in any order (e.g., alkoxy, alkenyloxy, carboxyl, sulfinyloxy, sulfonyloxy, and carbonylamino groups).

The positions of the linkage between the linking groups mentioned above and Rings A and B is also not limited and may be arbitrarily selected.

In Formula I, p represents an integer of 0 or 1. When p is 0, Rings A and B are linked together via a single bond.

In Formula I, q represents an integer of 1 or 2. When q is 2, the two occurrences of Ring B may be either identical to each other or different from each other.

In Formula I, r represents an integer of 1 or 2. When r is 2, the two occurrences of each of Ring B, L, p, and q may be either identical to each other or different from each other.

In Formula I, examples of substituents which aromatic hydrocarbon groups or aromatic heterocyclic groups as Ring A and Ring B and linking groups L may have include, although not limited to, groups selected from the following list.
*Halogen atom, hydroxyl, carboxyl, nitro, cyano, thiol, sulfinic acid, sulfonic acid, amino, amide, imino, and imide groups.
*Hydrocarbon, hydrocarbon oxy, hydrocarbon carbonyl (acyl), hydrocarbon oxycarbonyl, hydrocarbon carbonyloxy, hydrocarbon substitution amino, hydrocarbon substitution amino carbonyl, hydrocarbon carbonyl substitution amino, hydrocarbon substitution thiol, hydrocarbon sulfony, hydrocarbon oxysulfony, and hydrocarbon sulfonyloxy groups.

The "hydrocarbon" group used herein may be either aliphatic or aromatic, or any combination of these. The aliphatic hydrocarbon group used herein may be either chain or cyclic, or any combination of these. The chain hydrocarbon group used herein may be either straight chain or branched chain, or any combination of these. The cyclic hydrocarbon group may be either monocyclic or multicyclic, and the multicyclic group may be fused cyclic or bridged cyclic or spiro cyclic, or any combination of these. The "hydrocarbon" group may be either saturated or unsaturated, i.e., may contain one or more carbon-carbon double bonds. The term "hydrocarbon" group used herein encompasses alkyl, alkenyl, alkynyl, cycloalkyl, cycloalkenyl, cycloalkynyl, and aryl groups. The number of carbon atoms constituting the "hydrocarbon" group is not particularly limited, but in the case of chain hydrocarbon groups, it may typically be from 1 to 20, preferably from 1 to 15, more preferably from 1 to 12, and in the case of cyclic hydrocarbon groups, it may typically be from 3 to 20, preferably from 4 to 15, more preferably from 5 to 12. Unless otherwise indicated, one or more hydrogen atoms of such "hydrocarbon" groups may be replaced by any substituents, and one or more carbon atoms of such "hydrocarbon" groups may be replaced by any heteroatoms according to its valence. The type of heteroatom is not limited, but examples include nitrogen, oxygen, sulfur, phosphorus, and silicon atoms.
*Heterocyclic, heterocyclic oxy, heterocyclic carbonyl, heterocyclic oxycarbonyl, heterocyclic carbonyl oxy, heterocyclic amino, heterocyclic amino carbonyl, heterocyclic carbonyl-substituted amino, heterocyclic-substituted thiol, heterocyclic sulfonyl, heterocyclic oxysulfonyl, and heterocyclic sulfonyloxy groups.

The "heterocyclic" group used herein may be either saturated or unsaturated, i.e., may contain one or more carbon-carbon double bonds. The unsaturated heterocyclic group may be either aromatic or non-aromatic. The "heterocyclic" group may be either monocyclic or multicyclic, and the multicyclic group may be fused cyclic or bridged cyclic or spiro cyclic The number of ring atoms constituting the "heterocyclic" group is not particularly limited, but may typically be 3 or more, or 4 or more, or 5 or more, and typically 20 or less, or 15 or less, or 12 or less. The type of hetero atoms to be contained in the "heterocyclic" group is not particularly limited, examples including nitrogen atom, oxygen atom, sulfur atom, phosphorus atom, and silicon atom. The number of hetero atoms to be contained in the "heterocyclic" group is not particularly limited, but may typically be from 1 to 8, preferably from 1 to 5, more preferably from 1 to 3. When the "heterocyclic" group contains two or more hetero atoms, these hetero atoms may be either identical to each other or different from each other.

Insofar as the valency and physicochemical properties of any of the substituents mentioned above permit, the "substituents" according to the present invention may also encompass functional groups further substituted by one or more of the substituents mentioned above. When a certain functional group has substituents, the number of substituents is not limited as far as its valency and physicochemical properties permit. In addition, when two or more substituents are present, these substituents may be either identical to each other or different from each other.

In Formula I, when the aromatic hydrocarbon group or aromatic heterocyclic group as Ring A and/or Ring B has substituents, the number of substituents may be one, two or more. When the aromatic hydrocarbon group or aromatic heterocyclic group as Ring A and/or Ring B has two or more substituents, these substituents may be either identical to each other or different from each other.

In Formula I, when the aromatic hydrocarbon group or aromatic heterocyclic group as Ring A and/or Ring B has substituents, two or more of these substituents may be bonded together to form a ring that is fused to Ring A and/or Ring B.

According to an embodiment, the active pharmaceutical ingredients to be contained in the pharmaceutical composition of the present invention are selected from the compounds listed in Table 1 below.

**[Table 1-1]**

| No. | Compound name | Brand name (or development code) | No. | Compound name | Brand name (or development code) |
|---|---|---|---|---|---|
| 1 | Serabelisib | ART-001 | 21 | Pazopanib | Votrient |
| 2 | Axitinib | Inlvta | 22 | Regorafenib | Stivarga |
| 3 | Afatinib | Gilotrif | 23 | Lenvatinib | Lenvima |
| 4 | Erlotinib | Tarceva | 24 | Lapatinib | Tykerb |
| 5 | Osimertinib | Tagrisso | 25 | Ruxolitinib | Jakavi |
| 6 | Gefitinib | Iressa | 26 | Encorafenib | Braftovi |
| 7 | Dacomitinib | Vizimpro | 27 | Dabrafenib | Tafinlar |
| 8 | Imatinib | Gleevec | 28 | Vemurafenib | Zelboraf |
| 9 | Dasatinib | Sprycel | 29 | Ibrutinib | Imbruvica |
| 10 | Nilotinib | Tasigna | 30 | Trametinib | Mekinist |
| 11 | Bosutinib | Bosulif | 31 | Binimetinib | Mektovi |
| 12 | Ponatinib | Iclusig | 32 | Abemaciclib | Verzenio |
| 13 | Alectinib | Alecensa | 33 | Palbociclib | Ibrance |
| 14 | Entrectinib | Rozlytrek | 34 | Quizartinib | Vanflyta |
| 15 | Crizotinib | Xalkori | 35 | Gilteritinib | Xospata |
| 16 | Ceritinib | Zykadia | 36 | Miransertib | ARQ-092 |
| 17 | Lorlatinib | Lorbrena | 37 | Taselisib | GDC-0032 |
| 18 | Sunitinib | Sutent | 38 | Dactolisib | BEZ235 |
| 19 | Sorafenib | Nexavar | 39 | Apitolisib | GDC-0980 |
| 20 | Vandetanib | Caprelsa | 40 | Voxtalisib | XL765/SAR245409 |

**[Table 1-2]**

| No. | Compound name | Brand name (or development code) | No. | Compound name | Brand name (or development code) |
|---|---|---|---|---|---|
| 41 | Gedatolisib | PF-05212384/ PKI587 | 61 | | ZSTK474 |
| 42 | | PF-04691502 | 62 | | VS-5584 |
| 43 | Omipalisib | GSK-2126458 | 63 | | GSK2636771 |
| 44 | | LY294002 | 64 | | AZD8186 |
| 45 | Pilaralisib | XL-147 | 65 | | GSK1059615 |
| 46 | Buparlisib | BKM120 | 66 | Olanzapine | Zvprexa |
| 47 | Cabozantinib | XL-184/ BMS-907351 | 67 | Ondansetron | Zofran |
| 48 | Tofacitinib | Xeljanz | 68 | Topiroxostat | Uriadec |
| 49 | Idelalisib | Zydelig | 69 | Triamterene | Triteren |
| 50 | Cobimetinib | Cotellic | 70 | Nogitecan | Hycamtin |
| 51 | Alpelisib | Piqray | 71 | Prazosin | Minipress |
| 52 | Pictilisib | GDC-0941 | | | |
| 53 | Copanlisib | Aliquopa | | | |
| 54 | Fimepinostat | CUDC-907 | | | |
| 55 | Sonolisib | PX-866 | | | |
| 56 | Duvelisib | IPI-145 | | | |
| 57 | Umbralisib | TGR-1202 | | | |
| 58 | Tenalisib | RP6530 | | | |
| 59 | Samotolisib | LY3023414 | | | |
| 60 | | MEN1611 | | | |

According to an embodiment, the active pharmaceutical ingredient to be contained in the pharmaceutical composition of the present invention may be selected from serabelisib, alpelisib, afatinib, gefitinib, bosutinib, alectinib, palbociclib, taselisib, and copanlisib.

According to an embodiment, the active pharmaceutical ingredient to be contained in the pharmaceutical composition of the present invention may be serabelisib or alpelisib.

The pharmaceutical composition of the present invention may contain any one of the active pharmaceutical ingredients mentioned above, or any two or more in any combination at any ratios.

According to an embodiment, the active pharmaceutical ingredient to be contained in the pharmaceutical composition of the present invention may not maintain a stable crystalline structure in the pharmaceutical composition of the present invention but may be partially amorphous. According to a more preferred embodiment, even if the active pharmaceutical ingredient is an aromatic compound that can cause π-π stacking, the aromatic compound may preferably not cause π-π stacking in the pharmaceutical composition of the present invention, and each molecule may preferably exist in a separated state. The method for maintaining the active pharmaceutical ingredient in an amorphous state within the formulation will be explained below.

The content percentage of the active pharmaceutical ingredient in the pharmaceutical composition of the present invention may be determined according to various conditions, such as the type of the active pharmaceutical ingredient, indication, dosage form, and route of administration. According to an embodiment, the content percentage of the active pharmaceutical ingredient in the pharmaceutical composition of the present invention may be 2 mass % or more, or 5 mass % or more, or 10 mass % or more, or 15 mass % or more, and may be 40 mass % or less, or 35 mass % or less, or 30 mass % or less, or 25 mass % or less.

The pharmaceutical composition of the present invention may contain, in addition to the active pharmaceutical ingredient having a pH-dependent dissolution profile, one or more additional active pharmaceutical ingredients that do not have a pH-dependent dissolution profile, unless derogating from the intent of the present invention.

### Inorganic or organic acid:

The pharmaceutical composition of the present invention contains an inorganic or organic acid. The term inorganic or organic "acids" used herein means inorganic or organic compounds that can donate protons (H⁺ ) (so-called Brønsted acids). Although not bound by theory, it is assumed that such an acid existing in the pharmaceutical composition of the present invention may intervene between the molecules of the active pharmaceutical ingredient and thereby stabilize its dissolution profile regardless of pH.

According to an embodiment, the acids to be contained in the pharmaceutical composition of the present invention may be, although not limited to, acids having an acid dissociation constant (pKa value) of 4.2 or less, or 3.1 or less, or 2.2 or less. The term acid dissociation constant (pKa value) used therein means, unless otherwise stated, an acid dissociation constant in water and, in the case of polyvalent acids, an acid dissociation constant for a dissociation reaction that releases one hydrogen ion (so-called pKa₁ value). Acids with acid dissociation constants (pKa values) below the limits mentioned above may facilitate protonation of basic active pharmaceutical ingredients, and also solubilize active pharmaceutical ingredients with pH-dependent dissolution profiles.

According to an embodiment, the acids to be contained in the pharmaceutical composition of the present invention may be acids having a molecular mass within a predetermined range. Specifically, the molecular mass of the acid is not restricted, but may typically be between 30 and 200.

According to an embodiment, specific examples of acids to be contained in the pharmaceutical composition of the present invention may include, although not limited to, phosphoric acid, hydrochloric acid, citric acid, malic acid, tartaric acid, ascorbic acid, fumaric acid, succinic acid, aspartic acid, lactic acid, acetic acid, glutamic acid, and adipic acid. According to an embodiment, specific examples of acids may include phosphoric acid, hydrochloric acid, citric acid, malic acid, and tartaric acid. According to an embodiment, specific examples of acids to be contained in the pharmaceutical composition of the present invention may include phosphoric acid, citric acid, and tartaric acid. The pharmaceutical composition of the present invention may contain any one of these acids or any two or more in any combination at any ratios.

According to an embodiment, the acids to be contained in the pharmaceutical composition of the present invention may be compounds having two or more acidic functional groups. Examples of compounds having two or more acidic functional groups may include, although not limited to, compounds having two or more hydroxyl groups (e.g., phosphoric acid), compounds having two or more carboxyl groups (e.g., citric acid, malic acid, and tartaric acid), compounds having one or more hydroxyl groups and one or more carboxyl groups (e.g., lactic acid).

According to an embodiment, the content percentage of the acid in the pharmaceutical composition of the present invention may be, although not limited to, 5 mass % or more, or 10 mass % or more, or 15 mass % or more, and 50 mass % or less, or 45 mass % or less, or 40 mass % or less. According to an embodiment, the mass ratio of the inorganic or organic acid to the active pharmaceutical ingredient to be contained in the pharmaceutical composition of the present invention may be, relative to 1 mass part of the active pharmaceutical ingredient, 0.1 mass part or more, or 0.3 mass part or more, or 0.5 mass part or more, and 10 mass part or less, or 7.5 mass part or less, or 5 mass part or less of inorganic or organic acid.

The pharmaceutical composition of the present invention may contain, in addition to the inorganic or organic acid, one or more monovalent inorganic or organic acids, unless derogating from the intent of the present invention.

### Hydrophilic polymer:

The pharmaceutical composition of the present invention contains a hydrophilic polymer. Although not bound by theory, it is assumed that in the pharmaceutical composition of the present invention, in addition to the acid mentioned above, such a hydrophilic polymer may intervene between the molecules of the active pharmaceutical ingredient to prevent their re-aggregation and crystallization, thereby stabilizing its dissolution profile regardless of pH.

The term "hydrophilic polymer" used herein means a polymer that has the property of dissolving in water at room temperature. Specifically, it means a polymer that exhibits a viscosity of 1 mPa-s or more in 1 to 10% aqueous solution under normal pressure at 20°C.

According to an embodiment, the hydrophilic polymers to be contained in the pharmaceutical compositions of the present invention may include, although not limited to, those having carboxyl groups, hydroxyl groups, sulfo groups, amide groups, etc., as hydrophilic substituents. Such hydrophilic polymers can prevent reaggregation or crystallization of the active pharmaceutical ingredient through hydrophobic interactions between their molecules, and thereby contribute to stabilizing its dissolution profile.

According to an embodiment, specific examples of the hydrophilic polymers to be contained in the pharmaceutical composition of the present invention may include, although not limited to, polyvinyl alcohol, povidone, hypromellose, copovidone, hydroxypropylcellulose, polyvinyl alcohol-polyethylene glycol-graft copolymer, hypromellose phthalate and hypromellose acetate succinate. According to a specific embodiment, specific examples of the hydrophilic polymers to be contained in the pharmaceutical composition of the present invention may include polyvinyl alcohol, povidone, hypromellose, and hydroxypropylcellulose. The pharmaceutical composition of the present invention may contain any one of these hydrophilic polymers or any two or more in any combination at any ratios.

According to an embodiment, the content percentage of the hydrophilic polymer in the pharmaceutical composition of the present invention may be, although not limited to, 1 mass % or more, or 2 mass % or more, or 3 mass % or more, and 15 mass % or less, or 12.5 mass % or less, or 10 mass % or less. According to an embodiment, the mass ratio of the hydrophilic polymer to the active pharmaceutical ingredient may be, relative to 1 mass part of the active pharmaceutical ingredient, typically 0.02 mass part or more, or 0.05 mass part or more, or 0.1 mass part or more, and typically 5 mass part or less, or 3 mass part or less, or 2 mass part or less of the hydrophilic polymer.

The pharmaceutical composition of the present invention may contain, in addition to the inorganic or organic acid, one or more monovalent inorganic or organic acids, unless derogating from the intent of the present invention.

### Other ingredient:

The pharmaceutical composition of the present invention may further contain other ingredients. Specific examples of other ingredients may contain, although not limited to: excipients such as mannitol, erythritol, powder maltitol starch syrup, microcrystal cellulose, corn starch; disintegrants such as crospovidone, croscarmellose sodium, sodium starch glycolate, partially pregelatinized starch , carmellose calcium, low-substituted hydroxypropylcellulose; sweetening agents such as sucralose, aspartame, acesulfame potassium, saccharin sodium, monoammonium glycyrrhizinate, and thaumatin; and other additives such as fluidizers, colorants, and perfume. These ingredients may be selected and used as appropriate depending on the dosage form of the pharmaceutical composition of the present invention. The pharmaceutical composition of the present invention may contain any one of these ingredients alone, or any two or more in any combination at any ratios. For details of the ingredients that can be used in the pharmaceutical composition of the present invention, it is possible to consult the description of, e.g., University of the Sciences in Philadelphia, "Remington: The Science and Practice of Pharmacy, 20th EDITION", Lippincott Williams & Wilkins, 2000, as appropriate.

### Dosage form:

The dosage form of the pharmaceutical composition of the present invention is not limited and can be any dosage form that can be administered orally. Examples include various oral dosage forms listed in the Japanese Pharmacopoeia, such as tablets, capsules, granules, powders, oral liquids, syrups, and oral jellies. These dosage forms may be selected according to the type of the active pharmaceutical ingredient and indication.

According to an embodiment, the pharmaceutical composition of the present invention is in the form of dry syrup. The term "dry syrup" used herein means a subclass of "syrup" listed in the Japanese Pharmacopoeia, which is a granular or powdery dry solid formulation that can be reconstituted into syrup by adding water at the time of administration and dissolving or suspending it in water for use. Such dry syrup formulations are particularly advantageous when formulating active pharmaceutical ingredients (e.g., ART-001, alpelisib, etc.) that are mainly administered to children, because of their good storage and portability, ease of administration, and other advantages.

### [Method of Producing Oral Pharmaceutical Composition]

The method of producing the pharmaceutical composition of the present invention is not limited and can be made by any known method according to the dosage form. For example, depending on the desired dosage form, it can be produced by mixing the active pharmaceutical ingredient, inorganic or organic acid, and hydrophilic polymer, and optionally other ingredients using known formulation techniques, and carrying out additional processing such as granulation as appropriate. For example, in the case of granule formulations, a known granulation method such as dry granulation or wet granulation can be used to granulate the raw material mixture. In the case of tablets, the granulated raw material mixture obtained by known granulation methods can be compressed into tablets, and the resulting tablets can be coated as necessary. Alternatively, tablets may be manufactured by directly compressing the raw material mixture by a direct compression method without going through the granulation process.

According to an embodiment, when the pharmaceutical composition of the present invention is in the form of dry syrup, it can be produced using the active pharmaceutical ingredient, inorganic or organic acid, and hydrophilic polymer as raw materials, along with other optional ingredients as appropriate by a process comprising the following steps (also referred to as "the production method of the present invention" as appropriate):
- mixing the raw materials in an arbitrary mixing order;
- wet-granulating the mixture; and
- drying the wet-granulated product.

Some of the raw material components other than the active pharmaceutical ingredient may optionally be added later to the raw material mixture after wet granulation.

In the production method of the present invention, wet granulation of the raw material mixture containing the active pharmaceutical ingredient may serve to eliminate aggregation and crystallization of the molecules of the active pharmaceutical ingredient and help these molecules exist in an amorphous state in the pharmaceutical product.

The wet-granulating step will be specifically described below. The solvent used in the granulation process is not limited, and any known solvent can be used. Examples include water, ethanol, methanol, acetone, and dichloromethane. Preferred among these include water and ethanol from the viewpoint of handling. Any one of these solvents may be used alone, or any two or more may be used together in any combination at any ratios. The solvent is mixed with the raw material components, and the mixture is subjected to a granulation process. The granulation method used is not limited, and may be any known granulation method. Examples include extrusion granulation, spray-drying granulation, fluid-bed granulation, agitated granulation, etc. According to an embodiment, the granulation method is fluid bed granulation. Wet granulation machines are not limited, and any known granulator can be used, depending on the solvent and granulation method used. Examples include cylindrical extrusion granulators, rolling fluid bed granulators, spray-drying granulators, fluid bed granulation dryers, agitated granulators, etc. According to an embodiment, the granulator is a rolling fluidized bed granulator, fluidized bed granulator-dryer, etc.

In addition to the steps mentioned above, various well-known processes in the formulation field may be added as necessary, such as wet or dry milling of each raw material component, sieving of each raw material component or raw material mixture, coating of granules with a predetermined coating material, etc.

### [Usages of Oral Pharmaceutical Composition]

The pharmaceutical composition of the present invention may be orally administered to a subject for the purpose of treating, preventing, or modulating a disease or condition according to the type of the active pharmaceutical ingredient. The subject of administration of the pharmaceutical composition of the present invention is not particularly restricted, but examples include humans, mammals other than humans, and other animals. The dosage and frequency of administration of the pharmaceutical composition of the present invention are also not particularly restricted, but may be adjusted according to, for example, the type and efficacy of the active pharmaceutical ingredient and the species, age, weight, and condition of the subject to be administered. Two or more separate forms of the pharmaceutical compositions of the present invention containing two or more different active pharmaceutical ingredients may individually be administered to the same subject simultaneously or consecutively. Alternatively, one form of the pharmaceutical composition of the present invention containing one active pharmaceutical ingredient and another pharmaceutical composition containing another active pharmaceutical ingredient may individually be administered to the same subject simultaneously or consecutively. In particular, when a first active pharmaceutical ingredient with pH-dependent solubility and a second active pharmaceutical ingredient whose solubility is independent of pH are administered to the same subject, the pharmaceutical composition of the present invention formulated with the first active pharmaceutical ingredient with pH-dependent solubility and a conventional pharmaceutical composition formulated with the second active pharmaceutical ingredient with non-pH-dependent solubility may be combined and administered simultaneously or successively to the subject. In this case, the conventional pharmaceutical composition to be administered in combination with the pharmaceutical composition of the present invention may be administered orally or by another route.

### EXAMPLES

The present invention will be described in more detail in the following examples. However, these examples are only shown for convenience of explanation, and the present invention is not limited to these examples in any way.

### [1. Analysis of pH-dependent dissolution profile of ART-0011

The dissolution profile of ART-001 in various pH environments was tested by the following procedure. Excess amounts of ART-001 were added to 50 mM phosphate buffer (pH 1, 2, 7, and 8), citrate buffer (pH 3, 4, 5, and 6), boric acid buffer (pH 9), and bicarbonate buffer (pH 10), with the pH adjusted variously in the range of from pH 1 to pH 10 with a 6N HCl or 10N sodium hydroxide solution, and stirred at room temperature. When the dissolved state reached equilibrium, a fixed amount of each sample was taken. After centrifugation at 1000 rpm for 10 minutes, the supernatant was collected and measured by HPLC. The pH at the beginning and end of dissolution were also measured and recorded.

The results are shown in Table 2 below. As is clear from this table, ART-001 shows extremely high solubility under low pH conditions (e.g., pH 2.5 or lower) at room temperature (RT) (solubility 312 mcg/mL to approx. 10000 mcg/mL), whereas the solubility decreases rapidly as the pH increases, and little solubility is exhibited under high pH conditions (e.g., pH 6 or higher) (solubility 1.3 mcg/mL: about 4% when the solubility under low pH conditions is considered to be 100%). This indicates that ART-001 has an extremely pH-dependent dissolution profile.

### [Table 2]

**Table 2: Relationship between pH and solubility of ART-001 at room temperature (RT)**

| Start pH | End pH | Solubility (mcg/mL) |
|---|---|---|
| 1.04 | 1.33 | 1139 |
| 2.09 | 2.56 | 312 |
| 3.17 | 3.33 | 36.8 |
| 4.00 | 4.19 | 5.7 |
| 5.03 | 5.2 | 1.7 |
| 5.95 | 6.11 | 1.3 |
| 6.81 | 6.97 | 1.2 |
| 8.15 | 8.3 | 1.3 |
| 9.00 | 9.16 | 1.3 |
| 10.00 | 10.07 | 2.18 |

### [2. Preparation of ART-001 dry syrup formulations]

Dry syrup formulations of ART-001 were prepared according to the following procedure.

### Formulation A:

(1) 270 g of D-mannitol (Bussan Food Science Co., Ltd.) sieved through a 42M sieve was mixed with 30 g of microcrystalline cellulose (KG-1000, Asahi Kasei Corporation).
(2) 75 g of phosphoric acid (Kokusan Chemical Co., Ltd.) and 25 g of povidone (PLASDONE K-25, ASHLAND Co.) were dissolved in 600 g of purified water.
(3) 100 g of ART-001 (Carbogen Amcis AG) was added to the solution of (2) above and dispersed.
(4) The mixture of (1) above was fed into a fluidized bed granulation dryer (Model FD-MP-01D/SFP, Powrex Co.) and granulated by spraying the dispersion of (3) above.
(5) The granules obtained in (4) above were dried at 60°C.
(6) The granules obtained in (5) above were sieved through a 30M sieve.
(7) 0.25 g of the granulated material obtained in (6) above was mixed with 0.15 g of citric acid hydrate (Kokusan Chemical Co., Ltd.) to obtain a desired dry syrup formulation that corresponds to the pharmaceutical composition of the present invention.

### Formulation B:

(1) 270 g of D-mannitol (Bussan Food Science Co., Ltd.) sieved through a 42M sieve was mixed with 30 g of microcrystalline cellulose (KG-1000, Asahi Kasei Corporation).
(2) 75 g of phosphoric acid (Kokusan Chemical Co., Ltd.) and 25 g of povidone (PLASDONE K-25, ASHLAND Co.) were dissolved in 600 g of purified water.
(3) 100 g of ART-001 (Carbogen Amcis AG) was added to the solution of (2) above and dispersed.
(4) The mixture of (1) above was fed into a fluidized bed granulation dryer (Model FD-MP-01D/SFP, Powrex Co.) and granulated by spraying the dispersion of (3) above.
(5) The granules obtained in (4) above were dried at 60°C.
(6) The granules obtained in (5) above were sieved through a 30M sieve.
(7) 0.25 g of the granulated material obtained in (6) above was mixed with 0.0583 g of tartaric acid (L(+)-tartaric acid (Kokusan Chemical Co., Ltd.) and 0.035 g of L-glutamic acid hydrochloride (FujiFilm Wako Pure Chemicals Co., Ltd.) to obtain a desired dry syrup formulation that corresponds to the pharmaceutical composition of the present invention.

### Formulation C:

(1) 48 g of microcrystalline cellulose (KG-1000, Asahi Kasei Corporation) was mixed with 32 g of polyvinyl alcohol (partially-saponified) (GOHSENOL EG-05PW, Mitsubishi Chemical Corporation).
(2) 160 g of D-mannitol (Bussan Food Science Co., Ltd.) sieved through a 42M sieve was mixed with the mixture in (1) above.
(3) 120 g of phosphoric acid (Taihei Chemical Industry Co., Ltd.) and 40 g of povidone (PLASDONE K-25, ASHLAND) were dissolved in 960 g of purified water.
(4) 160 g of ART-001 (Carbogen Amcis AG) was added to the solution of (3) above and dispersed.
(5) The mixture of (2) above was fed into a fluidized bed granulation dryer (Model FD-MP-01D/SFP, Powrex Co.) and granulated by spraying the dispersion of (4) above.
(6) The granules obtained in (5) above were dried at 60°C.
(7) The dried granules obtained in (6) above were sieved through a 30M sieve to obtain a desired dry syrup formulation that corresponds to the pharmaceutical composition of the present invention.

### [3. Evaluation of ART-001 dry syrup formulations]

The dry syrup formulations of ART-001 prepared by the procedures described above were administered to dogs by the procedure detailed below, and the pharmacokinetics of ART-001 in blood was observed. As a control, a preparation in which ART-001 was suspended in 0.5% aqueous solution of methylcellulose (MC; Shin-Etsu Chemical Co., Ltd. METOLOSE, SM-100) was prepared, and was administered to dogs in a similar manner, and the pharmacokinetics of ART-001 in blood was observed.

Specifically, a total of five administration tests were conducted on six male dogs of 5- to 6-year-old obtained from Kitayama Labes Co. with a rest period of at least one week. Thirty minutes before the start of each formulation administration, each individual was administered intramuscularly with pentagastrin (Sigma-Aldrich) in Test 1 to adjust the intragastric pH to storngly acidic, or was administered intravenous with famotidine (Fujifilm Wako Pure Chemicals Co., Ltd.) in Tests 2 through 5 to adjust the intragastric pH to neutral to slightly alkaline. The intragastric pH of each individual in each group was then measured with pH test paper immediately prior to the start of administration of each formulation. Each individual was administered with 0.5% MC suspension of ART-001 as the control in Test 1 and Test 2, and with ART-001 dry syrup formulations A, B and C in Tests 3, 4, and 5, respectively, at a dose of 4 mg/kg. After administration, blood was collected over time from the radial skin vein of each individual in each group, and the concentration of ART-001 in the plasma (µ g/mL) was measured by HPLC. The results are shown in Table 3 below.

In Test 1, in which 0.5% MC suspension of ART-001 was administered to adjust the intragastric pH to strongly acidic (approx. pH 2), the plasma ART-001 concentration reached about 1.4 µg/mL at about 0.5 hours after administration, and then gradually decreased. On the other hand, in Test 2, in which the intragastric pH was adjusted to neutral to slightly alkaline (approx. pH 8 to 9), the plasma ART-001 concentration hardly increased after administration. This indicates that the intragastric pH has a significant effect on the absorption of ART-001.

On the other hand, in Tests 3 to 5, in which the ART-001 dry syrup formulations A to C was administered, the maximum plasma ART-001 concentration (Cₘₐₓ) and the area under the blood concentration curve (AUC 0-24h) were similar to those of Test 1, despite the fact that the intragastric pH was adjusted to neutral to slightly alkaline (approx. pH 8 to 9). These results indicate that the ART-001 dry syrup formulations A to C, which correspond to the pharmaceutical compositions of the present invention, can achieve substantially the same absorption profile even when the intragastric pH is neutral to slightly alkaline (approx. pH 8 to 9) as when the intragastric pH is strongly acidic (approx. pH 2).

### [Table 3]

**Table 3: Plasma ART-001 concentration over time after administration**

| | API formulation | Intragastric treatment Intragastric pH | Cₘₐₓ (µg/mL) | Tₘₐₓ (h) | AUC_{0.24h} (µg/mL) | MRT (h) |
|---|---|---|---|---|---|---|
| Test 1 | ART-001 0.5 %MC suspension | Pentagastrin pH 2 | 1.427 ±0.615 | 0.46 ±0.29 | 2.932 ±2.064 | 1.68 ±0.58 |
| Test 2 | ART-001 0.5%MC suspension | Famotidine pH 8 to 9 | 0.009 ±0.004 | 0.50 ±0.27 | 0.026 ±0.027 | 4.83 ±7.76 |
| Test 3 | ART-001 dry syrup formulation A | Famotidine pH 9 | 1.540 ±0.559 | 0.38 ±0.14 | 4.352 ±1.983 | 2.42 ±0.71 |
| Test 4 | ART-001 dry syrup formulation B | Famotidine pH 9 | 1.669 ±0.630 | 0.46 ±0.10 | 3.907 ±2.565 | 2.12 ±0.74 |
| Test 5 | ART-001 dry syrup formulation C | Famotidine pH 8 to 9 | 1.476 ±0.585 | 0.67 ±0.38 | 3.366 ±1.803 | 1.95 ±0.47 |

### [4. Analysis of pharmacokinetics of ART-001 dry syrup formulations in blood]

A dry syrup formulation of ART-001 (the pharmaceutical composition of the present invention) prepared by the procedure described above were administered as a single or repeated dose to healthy adult males, and the subsequent pharmacokinetics of ART-001 in blood were studied.

In the single-dose study, the dry syrup formulation of ART-001 was dissolved in 100 mL of water at ART-001 doses of 50 mg, 100 mg, 200 mg, 300 mg, or 400 mg, and each solution was administered as a single oral dose to six healthy adult males. Blood samples were collected over time from before administration to 48 hours after administration, and the plasma ART-001 concentration (ng/mL) was measured by HPLC. In the repeated-dose study, the dry syrup formulation of ART-001 was dissolved in 100 mL of water at an ART-001 dose of 100 mg, and the solution was administered orally once daily for 7 days to six healthy adult males, and blood samples were taken over time.

The results of the single-dose study are shown in Figure 1 and Table 4. After administration of the dry syrup formulations, ART-001 was rapidly absorbed and reached its maximum plasma concentration (Cₘₐₓ) approximately 1 hour after administration, followed by a gradual decrease (Figure 1). The Cₘₐₓ and the area under the blood concentration curve (AUCₗₐₛₜ) of ART-001 increased with dose of ART-001 (Table 4). The coefficient of variation (%CV), a measure of the variability of plasma concentrations, ranged from 3 to 22% for Cₘₐₓ and from 16 to 34% for AUCₗₐₛₜ.

### [Table 4]

**Table 4: Cₘₐₓ and AUCₗₐₛₜ in healthy adults after single-dose administration of ART-001 dry syrup formulations**

| Dosage | N | Cₘₐₓ, ng/mL (%CV) | AUCₗₐₛₜ, ng•h/mL (%CV) |
|---|---|---|---|
| 50 mg | 6 | 1033 ± 129 (13) | 9844 ± 3318 (34) |
| 100 mg | 6 | 2560 ± 567 (22) | 28393 ± 7340 (26) |
| 200 mg | 6 | 4913 ± 167 (3) | 61681 ± 21171 (34) |
| 300 mg | 6 | 7523 ± 699 (9) | 119765 ± 19066 (16) |
| 400 mg | 6 | 8282 ± 1070 (13) | 157400 ± 42215 (27) |

| | | | |
|---|---|---|---|
| Mean ± S.D. | | | |

The results of the repeated-dose study are shown in Figure 2 and Table 5. On Day 5 of administration of the dry syrup formulation, the trough value of the plasma ART-001 concentration reached steady state, and the accumulation rate of ART-001 after 7 days of administration in terms of AUC₀₋₂₄ₕ was 2.4-fold (Figure 2, Table 5). In addition, the %CV on Day 7 of administration was 19% for Cₘₐₓ and 32% for AUC₀₋₂₄ₕ, which was equivalent to that on Day 1 of administration.

### [Table 5]

**Table 5: Cₘₐₓ and AUC₀₋₂₄ₕ in healthy adults after 7-day repeated administration of ART-001 dry syrup formulations**

| Dosage | Administration date | N | Cₘₐₓ, ng/mL (%CV) | AUC₀₋₂₄ₕ ng•h/mL (%CV) |
|---|---|---|---|---|
| 100 mg/day | Day 1 | 6 | 1318±198 (15) | 16029±4188 (26) |
| | Day 7 | 6 | 2128±407 (19) | 37740±12009 (32) |

| | | | | |
|---|---|---|---|---|
| Mean ± S.D. | | | | |

These results indicate that when ART-001, an example of an active pharmaceutical ingredient with a pH-dependent dissolution profile, is administered using a dry syrup formulation which corresponds to the pharmaceutical composition of the present invention, more stable pharmacokinetics with less inter-individual variability can be obtained than when administered using previously-reported capsules or tablets (references: Juric et al., Clin. Cancer Res., (2017), 23[17]:5015-5023, Patel et al., Clin. Pharmacol. Drug Dev., (2019), 8[5]:637-646). The tight and stable pharmacokinetics obtained with such dry syrup formulations serve to facilitate dose selection to obtain the expected effective blood exposure and avoid side effects due to high exposure.

### INDUSTRIAL APPLICABILITY

The present invention can be widely used in the field of pharmaceutical formulations, especially for administering various active pharmaceutical ingredients with pH-dependent dissolution profiles. Its use is therefore of great value.

## Claims

1. An oral pharmaceutical composition comprising: an active pharmaceutical ingredient having a pH-dependent dissolution profile; an inorganic or organic acid; and a hydrophilic polymer.

2. The oral pharmaceutical composition according to claim 1, wherein the active pharmaceutical ingredient is an aromatic compound having a π-conjugated system.

3. The oral pharmaceutical composition according to claim 1 or 2, wherein the active pharmaceutical ingredient is a compound having a structure represented by Formula I. 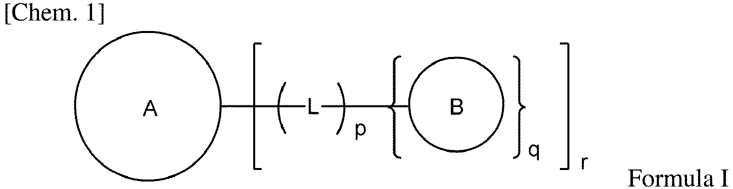 wherein
Ring A represents a 5- to 16-membered monocyclic or fused bicyclic or tricyclic aromatic hydrocarbon group or aromatic heterocyclic group that may have one or more substituents,
Ring B represents a 5- to 12-membered monocyclic or fused bicyclic aromatic hydrocarbon group or aromatic heterocyclic group that may have one or more substituents,
L represents a divalent or trivalent linking group that may have one or more substituents,
p represents an integer of 0 or 1,
q represents an integer of 1 or 2, and
r represents an integer of 1 or 2.

4. The oral pharmaceutical composition according to any one of claims 1 to 3, wherein the active pharmaceutical ingredient is one or more compounds selected from ART-001 (serabelisib), alpelisib, afatinib, gefitinib, bosutinib, alectinib, palbociclib, taselisib, and copanlisib.

5. The oral pharmaceutical composition according to any one of claims 1 to 4, wherein the ratio of the solubility at pH 6 to the solubility at pH 2.5 of the active pharmaceutical ingredient is 50% or less.

6. The oral pharmaceutical composition according to any one of claims 1 to 5, wherein the inorganic or organic acid is one or more acids selected from phosphoric acid, hydrochloric acid, citric acid, malic acid, tartaric acid, ascorbic acid, fumaric acid, succinic acid, aspartic acid, lactic acid, acetic acid, glutamic acid, and adipic acid.

7. The oral pharmaceutical composition according to any one of claims 1 to 6, wherein the hydrophilic polymer is one or more polymers selected from polyvinyl alcohol, povidone, hypromellose, copovidone, hydroxypropylcellulose, polyvinyl alcohol-polyethylene glycol-graft copolymer, hypromellose phthalate, and hypromellose acetate succinate.

8. The oral pharmaceutical composition according to any one of claims 1 to 7, wherein the content percentage of the active pharmaceutical ingredient is from 2 to 40 mass %

9. The oral pharmaceutical composition according to any one of claims 1 to 8, wherein the content ratio of the inorganic or organic acid to one mass part of the active pharmaceutical ingredient is from 0.1 to 10 mass parts.

10. The oral pharmaceutical composition according to any one of claims 1 to 9, wherein the content ratio of the hydrophilic polymer to one mass part of the active pharmaceutical ingredient is from 0.02 to 5 mass parts.

11. The oral pharmaceutical composition according to any one of claims 1 to 10, which is in the form of a dry syrup.

12. A method of producing an oral pharmaceutical composition according to any one of claims 1 to 11, comprising: wet-granulating a mixture containing the active pharmaceutical ingredient, the inorganic or organic acid, and the hydrophilic polymer, and drying the granulated product.

13. An oral pharmaceutical composition obtainable by the method according to claim 12.
